# EUROPEAN PATENT SPECIFICATION

(11) **EP 3 000 822 B1**
(45) Date of publication and mention of the grant of the patent: **31.10.2018**
(21) Application number: 15020002.0
(22) Date of filing: 08.01.2015
(51) Int. Cl.: C07K 1/04, C07K 14/21

(54) **A novel method of pseudofactin synthesis**
Neuartiges Verfahren zur Pseudofactinsynthese
Nouveau procédé de synthèse de pseudofactine

(30) Priority: 25.09.2014 PL 40959514
(43) Date of publication of application: 30.03.2016
(73) Proprietor: Uniwersytet Wroclawski, 50-137 Wroclaw (PL)
(72) Inventor: Krasowska, Anna, 51-114 Wroclaw (PL); Lukaszewicz, Marcin, 51-351 Wroclaw (PL); Grzywacz, Daria, 80-298 Gdansk (PL); Kamysz, Wojciech, 83-210 Zblewo (PL)
(74) Representative: Kondrat, Mariusz

(56) References cited:
- US-A- 6 008 058
- MAÏDER PAGADOY ET AL: "Solid-Phase Synthesis of Surfactin, a Powerful Biosurfactant Produced by Bacillus subtilis, and of Four Analogues", INTERNATIONAL JOURNAL OF PEPTIDE RESEARCH AND THERAPEUTICS ; FORMERLY KNOWN AS LETTERS IN PEPTIDE SCIENCE, KLUWER ACADEMIC PUBLISHERS, DO, vol. 11, no. 3, 1 September 2005 (2005-09-01), pages 195-202, XP019287629, ISSN: 1573-3904
- JANEK TOMASZ ET AL: "Identification and characterization of biosurfactants produced by the Arctic bacteriumPseudomonas putidaBD2", COLLOIDS AND SURFACES. B, BIOINTERFACES, ELSEVIER, AMSTERDAM, NL, vol. 110, 14 May 2013 (2013-05-14), pages 379-386, XP028573153, ISSN: 0927-7765, DOI: 10.1016/J.COLSURFB.2013.05.008
- JANEK T ET AL: "Isolation and characterization of two new lipopeptide biosurfactants produced by Pseudomonas fluorescens BD5 isolated from water from the Arctic Archipelago of Svalbard", BIORESOURCE TECHNOLOGY, ELSEVIER BV, GB, vol. 101, no. 15, 1 August 2010 (2010-08-01), pages 6118-6123, XP027018557, ISSN: 0960-8524 [retrieved on 2010-03-19]
- TOMASZ JANEK ET AL: "Antiadhesive activity of the biosurfactant pseudofactin II secreted by the Arctic bacterium Pseudomonas fluorescens BD5", BMC MICROBIOLOGY, BIOMED CENTRAL LTD, GB, vol. 12, no. 1, 23 February 2012 (2012-02-23), page 24, XP021117894, ISSN: 1471-2180, DOI: 10.1186/1471-2180-12-24
- JANEK TOMASZ ET AL: "Lipopeptide Biosurfactant Pseudofactin II Induced Apoptosis of Melanoma A 375 Cells by Specific Interaction with the Plasma Membrane", PLOS ONE, vol. 8, no. 3, March 2013 (2013-03), XP002736744, ISSN: 1932-6203
- KONNO HIROYUKI ET AL: "Synthesis and antifungal activities of cyclic octa-lipopeptide burkholdine analogues", BIOORGANIC & MEDICINAL CHEMISTRY LETTERS, PERGAMON, AMSTERDAM, NL, vol. 23, no. 14, 14 May 2013 (2013-05-14), pages 4244-4247, XP028573308, ISSN: 0960-894X, DOI: 10.1016/J.BMCL.2013.04.091
- MCMURRAY J S: "SOLID PHASE SYNTHESIS OF A CYCLIC PEPTIDE USING FMOC CHEMISTRY", TETRAHEDRON LETTERS, PERGAMON, GB, vol. 32, no. 36, 1 January 1992 (1992-01-01), pages 7679-7682, XP002910483, ISSN: 0040-4039, DOI: 10.1016/0040-4039(91)80563-L

## Description

The presented disclosure relates to the solid phase peptide synthesis (SPPS) of pseudofactin, a biosurfactant lipopeptide with surface tension lowering properties.

*Pseudomonas fluorescens* BD5 strain, producing and secreting biosurfactant pseudofactin II, was isolated from water samples collected by the team of the Faculty of Earth Sciences and Environmental Science, University of 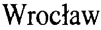 , at Spitsbergen in 2005. In the laboratory of the Department of Biotransformation, Faculty of Biotechnology, University of 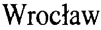 , the strain was identified using the API 20E test used for the identification of *Enterobacteriaceae* and by sequencing of the 16S rRNA subunit.

A 968 bp sequence was compared with the sequences of rDNA deposited in GenBank (http://www.ncbi.nlm.nih.gov/BLAST/). The isolated strain is a Gram-negative bacteria, aerobic and rod-shaped. The optimal temperature for the cultivation of the strain on MSM substrate with 2% glucose is 28°C. The strain produces cytochrome oxidase and utilizes citrate, but does not produce β galactosidase, ornithine decarboxylase, urease, tryptophan deaminase, indole or acetoin (Janek T., tukaszewicz M., Rezanka T., Krasowska A., (2010) Isolation and characterization of two new lipopeptide biosurfactants produced by Pseudomonas fluorescens BD5 isolated from water from the Arctic Archipelago of Svalbard. Bioresource Technology 101, 6118-6123).

*P. fluorescens* BD5 produces two variants of pseudofactin (known as PF I and PF II). These are cyclic lipopeptides that differ in the last amino acid at the C-terminus; valine for PF I and leucine for PF II. In this study PF II was selected for all experiments, as the amount produced was about 12 times greater than that of PF I.

PF II lowers the surface tension of water from 72 to 31.5 mN/m. Critical micelle concentration (CMC) was determined at 72 mg/l in water and was 2 times higher when measured in RPMI medium. The emulsifying properties of PF II are from 10% to 45% better than those exhibited by the surfactants Tween 20 and Triton X-100 (Janek T., tukaszewicz M., Rezanka T., Krasowska A., (2010) Isolation and characterization of two new lipopeptide biosurfactants produced by Pseudomonas fluorescens BD5 isolated from water from the Arctic Archipelago of Svalbard. Bioresource Technology 101, 6118-6123).

PF II reduces the adhesion on three different types of surfaces (glass, polystyrene, silicone) of Gram-negative bacteria strains such as: *Escherichia coli, Proteus mirabilis,* Gram-positive bacteria: *Enterococcus faecalis, Enterococcus hirae, Staphylococcus epidermidis, Staphylococcus aureus* and yeast: *Candida albicans.* Bacterial adhesion was inhibited by 36-90% and yeast adhesion by 92-99% in 0.5 mg/ml of PF II. In addition, PF II detaches already existing biofilms by 26-70%. Thanks to these properties PF II can be used as a disinfectant or agent for coating various surfaces, protecting them against microbial colonization (Janek T., 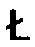 ukaszewicz M., Krasowska A. (2012) Antiadhesive activity of pseudofactin - biosurfactant secreted by arctic bacteria *Pseudomonas fluorescens* BD5. BMC Microbiology, 12:24, DOl: 10.1186/1471-2180-12-24).

PF II induces apoptosis in A375 melanoma cells without being cytotoxic to normal human dermal fibroblasts in similar experimental conditions. The mechanism of apoptosis induction by PF II seems to be different than in other biosurfactants described in literature. PF II permeabilizes the membrane by interacting with it in the form of micelles (active concentration - above CMC). Membrane permeabilization was found by measuring the LDH released into the medium (Janek T., Krasowska A., Radwanska A. Lukaszewicz M., (2013) Lipopeptide biosurfactant pseudofactin II induced apoptosis of melanoma A 375 cells by specific interaction with the plasma membrane, PlosOne, 8(3) doi:10.1371/journal.pone.0057991).

Skin tests were performed on an adult healthy male man and no allergenic activity of PF II was observed.

The existing methods for extracting pseudofactin II are not able to meet the demand for this substance and do not fulfil purity requirements for the production of medicines, in particular in the registration procedure, which requires the isolation and identification of all the impurities present in the product and their effects on the human body.

Furthermore, there is the risk of losing the strain capable of producing PF II, for example through mutation.

The aim of the invention therefore is PF II synthesis, with high performance and high product purity.

It was found that the addition of DIPEA (0.2 eq) to the reaction mixture during the internal cyclization of the peptide (consisting of the creation of an ester bond between the hydroxyl group of the side chain of L-threonine and the carboxyl group of L-leucine) resulted in a product of very high purity and yield.

The invented method of pseudofactin synthesis comprises the following steps:
a) solid phase synthesis of the peptide Palm-Gly-Ser(tBu)-Thr-Leu-Leu-Ser(tBu)-Leu-Leu-OH by the carbodiimide method wherein the solid phase is preferably the 2-chlorotrityl resin
b) cleaving the peptide from the resin while maintaining the tBu protecting group on the side chain of L-serine
c) internal cyclization of the peptide by the formation of an ester bond between the hydroxyl group on the side chain of L-threonine and the carboxyl group of L-leucine, in the presence of the catalyst mixture: diisopropylethylamine (DIPEA) 4-dimethylaminopyridine and *N*,*N'*-diisopropylocarbodiimide in DMF and DCM solvents.
d) removing the protecting groups located on the side chain of L-serine
e) purification of the peptide obtained in step d)
wherein the yield of the synthesis is up to 68 %.
Advantageously, step a) according to the invention comprises:
i. attachment of the first amino acid Fmoc-L-Leu-OH to the resin,
ii. synthesis of the linear peptide Palm-Gly-Ser(tBu)-Thr-Leu-Leu-Ser(tBu)-Leu-Leu- OH
Advantageously, cleavage of the peptide from the resin while maintaining the protecting groups of the side chain of L-serine is performed by a solution containing 50% acetic acid and 50% toluene.
PF II was synthesized manually by *(solid phase peptide synthesis) (SPPS)* with the use of the Fmoc/tBu strategy.

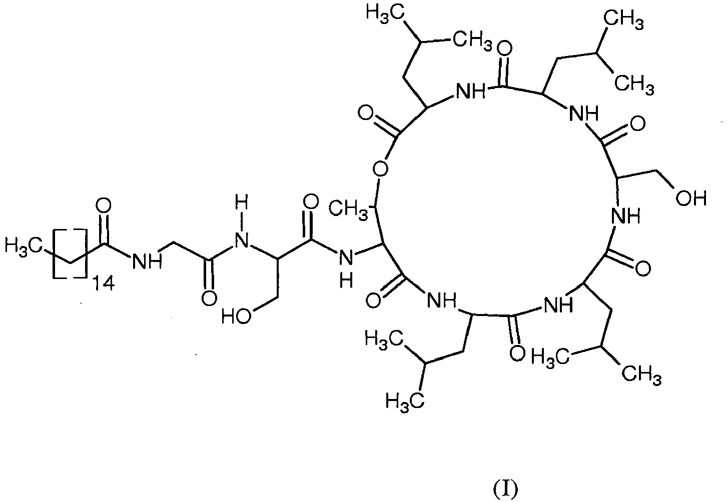

The synthesis uses only the L-configuration amino acids. The α-amino group of all amino acids is protected by the Fmoc group, while the side chain of L-serine was blocked by the tert-butyl protecting group (tBu). The peptide is modified by a saturated fatty acid with sixteen carbon atoms (palmitic acid).

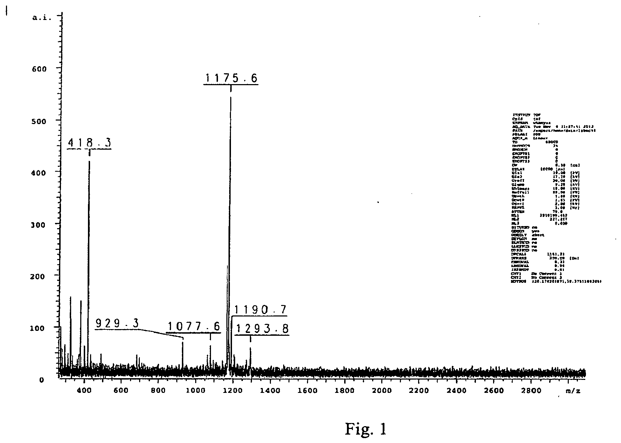

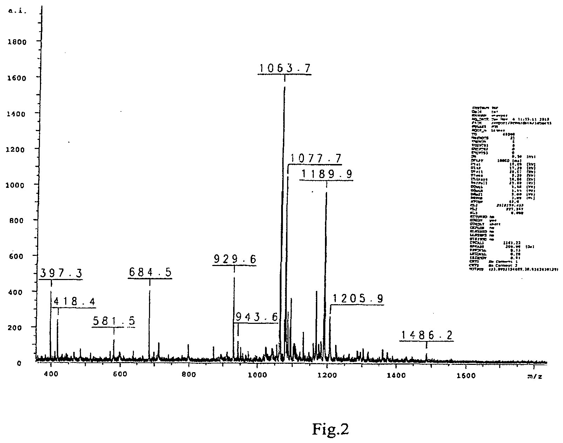

The structure obtained during pseudofactin synthesis is shown in the Figures below. Figure 1 shows the mass spectrum of the cyclic lipopeptide - pseudofactin with the protecting groups in the side chain, and Figure 2 includes a mass spectrum of the cyclic lipopeptide - pseudofactin without the protecting groups.

The method of the invention and the use of the compounds obtained are illustrated in the following examples.

### List of abbreviations:

Arg - Arginine
Gly - Glycine
DIPEA- Diisopropylethylamine
DIC - *N,N'*-Diisopropylcarbodiimide
DMAP - 4-Dimethylaminopyridine
DMF - *N,N*-Dimethylformamide
DCM - Dichloromethane
Fmoc - 9-fluorenylmethoxycarbonyl
Leu - Leucine
Palm - Palmitic acid
Pbf- 2,2,4,6,7-pentamethyldihydrobenzofuran-5-sulfonyl
Ser - Serine
tBu- Tert-butyl
TFA - Trifluoroacetic acid
Thr - Threonine

### Example 1. Description of the pseudofactin synthesis

1. 0.5 g of 2-chlorotrityl resin was added to the reaction vessel. The resin was swelled in dichloromethane for 60 min. After filtration of the solvent, 0.7 mmol of Fmoc-L-Leu-OH and 1.5 mmol of DIPEA were added. The solution was stirred on a shaker overnight. After checking the loading of the amino acid (0.99 mmol/g), the solvents were filtered. Then the synthesis of the linear peptide Palm-Gly-Ser(tBu)-Thr-Leu-Leu-Ser(tBu)-Leu-Leu-OH was performed in accordance with a standard procedure which comprises:
   a) deprotection of the amino function with 20% piperidine / DMF
   b) resin wash cycle (DMF, DCM)
   c) acylation (coupling) using an equimolar mixture of the protected amino acids DIC and Oxyma Pure (Fmoc-AA : DIC: Oxyma Pure, 1:1:1). Additional amino acid derivatives were attached using a 2-fold molar excess relative to the loading of the resin, the mixture was stirred for 60 min. The progress of acylation was tested by a chloranil test.
2. In order to cleave the peptide from the resin while maintaining the side-chain protecting group for L-serine, 10 ml of a solution containing 50% acetic acid and 50% toluene was prepared. The peptide-resin was flooded with the solution and stirred on a magnetic stirrer for 3 h at 37 °C. Then the solution was filtered into a round bottom flask and the solvents were evaporated. A few ml of acetic acid was added to the flask and then frozen before lyophilization. The obtained compound was analyzed by TLC. Analysis conditions were as follows: DCM: methanol (5:1; v:v), the TLC plate was developed in iodine vapor. MALDI-TOF mass analysis was also performed (signals found: m/z 1175.6 [M+23]⁺, 1190.7 [M+39]+). The results are shown in FIG. 1.
3. For the next step (internal cyclization), 30 mg (0.026 mmol) of the obtained peptide was weighed, dissolved in 10 ml of DMF and DCM, and then 0.33 mg (0.1 eq.) DMAP, 1 µl (0.2 eq.) DIPEA and 4.5 µl of DIC (1 eq.) were added. The solution was stirred on a magnetic stirrer for 4h. The reaction and the purity of the resulting products were analyzed by thin layer chromatography (TLC). After cyclization, the solvents were evaporated in a rotary vacuum. Separation of the resulting mixture of products was performed by column chromatography. The following arrangement of solvents was used as the mobile phase: DCM:MeOH (5:1; v:v).
4. The last step was to remove the protecting groups on the side chain of the obtained peptide. The lyophilized compound was dissolved in a mixture of TFA:H₂O (19:1, 700 µl) and stirred on a magnetic stirrer for 60 min. After that time, the solvents were evaporated, water was added and the mixture was lyophilized. The resulting compound was purified by column chromatography. The column was filled with silica gel (Kiesgel MN-60) with a particle size <0.08 nm (Merck). 10 g of silica gel was used per 0.1 g of the reaction product mixture. The following system of solvents was used as the mobile phase: DCM: MeOH: AcOH (5:1: 0.5; v:v:v). The obtained oily product was purified, obtaining 12 mg of pure peptide (68% yield). The procedure was confirmed by mass analysis (signals m/z: 1063.7 [M+23]+ and 1077.7 [M+39]+). The results are shown in FIG. 2 .

### Example 2.

Microorganisms were cultured on either liquid or solid media prepared in deionized water. The experiment was performed using the following bacteria: *Escherichia coli, Enterococcus faecalis, Enterococcus hirae, Staphylococcus aureus* and *Staphylococcus epidermidis,* cultured for 24 h in LB medium at 37°C, and the yeast *Candida albicans,* grown in YNB medium at 28°C. The strains were taken from a frozen state (-80°C) and inoculated on LB medium (bacteria) and YPG medium (yeast). After 24 or 48 hours of incubation at 28°C or 37°C the cultures were prepared for further experiments. 5 ml of liquid LB or YNB medium was inoculated with bacteria or yeast and incubated at 28°C or 37°C for 22-24 hours.

Bacterial and yeast biofilms were formed on plastic Thermanox discs with a diameter of 13 mm, on glass coverslips 10 mm in diameter, and on cut segments of silicone catheters, arranged in a 24-well plate. The prepared wells were loaded with 500 ml of bacterial suspension in LB and yeast in RPMI-1640 with an optical density OD₆₀₀=0.01 and 0.25 mg/ml of pseudofactin II. Control wells contained no pseudofactin II.

After 24 hours of incubation at 37°C, biofilms were gently rinsed in PBS and incubated for 30 minutes at 37°C in 1 ml of PBS containing 0.6% of Live/Dead BacLight viability kit (Molecular Probes, Eugene, OR) for bacteria, and Concanavalin A-Alexa Fluor 488 conjugate (Molecular Probes) at 25 µg/ml for yeast. After that time the solutions of the dyes were removed, and the biofilm was rinsed twice with PBS. The preparations on the slides were then observed under a scanning confocal microscope Olympus Fluoview 500 (Olympus Optical Co.Ltd., Japan). Preparations were analyzed and photographed.

In experiments on microbial colonization of catheters in the presence of pseudofactin II, 10 µl inoculum of the tested strains was transferred to 1000 µl of fresh media, LB and YNB. Then 1000 µl of pseudofactin II solution was added to the suspensions, which resulted in a concentration in the tested samples of 0.25 mg/ml. In order to prepare biofilm on catheters we placed 4 cm long segments of catheters in the cell suspension and incubated for 24 h at 37°C. After 24 h, the portions of catheters were flushed with distilled water. After rinsing, the catheters were stained with 0.1% crystal violet solution for 20 minutes and then washed three times with distilled water, then allowed to dry at room temperature. In another variant of the experiment, we used dynamic conditions, with a flow of 50 ml/h using a peristaltic pump. That experiment was performed twice.

In a parallel experiment, fragments of catheters were placed in sterile tubes with 2 ml of pseudofactin II solution in PBS at a concentration of 0.25 mg/ml and incubated for 2 h at 37°C. After 2 hours, the catheters were rinsed twice with PBS. Then, after the addition of 2 ml of inoculum the catheters were incubated at 37°C for 24 hours. The experiment was carried out twice, and staining with 0.1% crystal violet was performed as in the previous paragraph.

The obtained results show a strong inhibitory effect of pseudofactin II on the formation of bacterial and yeast biofilm under aerobic conditions. The pseudofactin II added to the samples resulted in a very strong reduction (from 90 to 95%) of biofilm formation on plastic, glass and silicone surfaces.

## Claims

1. A method of pseudofactin synthesis comprising the following steps:
a) solid phase synthesis of the peptide Palm-Gly-Ser(tBu)-Thr-Leu-Leu-Ser(tBu)-Leu-Leu-OH using the carbodiimide method wherein the solid phase is preferably the 2-chlorotrityl resin
b) cleavage of the peptide from the resin while maintaining the tBu protecting group on the side chain of L-serine
c) internal cyclization of the peptide by the formation of an ester bond between the hydroxyl group on the side chain of L-threonine and the carboxyl group of L-leucine, in the presence of the catalyst mixture: diisopropylethylamine (DIPEA), 4-dimethylaminopyridine and *N,N*'-diisopropylocarbodiimide in DMF and DCM solvents.
d) removing the protecting groups located on the side chain of L-serine
e) purification of the peptide obtained in step d)
wherein the yield of the synthesis is up to 68 %.

2. The method of pseudofactin synthesis according to claim 1, wherein step a) comprises the following:
i. attachment of the first amino acid to the resin, wherein the amino acid was Fmoc-L-Leu.
ii. synthesis of the linear peptide Palm-Gly-Ser(tBu)-Thr-Leu-Leu-Ser(tBu)-Leu-Leu-OH

3. The method of pseudofactin synthesis according to claim 1, **characterised in that**, the cleavage of the peptide from the resin while maintaining the protecting group in the side chain of L-serine is performed by using a solution containing 50% acetic acid and 50% toluene.

## Patentansprüche

1. Verfahren zur Synthese der Pseudofactin, das folgende Stufen umfasst:
a) Festphasensynthese des Peptids Palm-Gly-Ser (tBu)-Thr-Leu-Leu-Ser (tBu)-Leu-Leu-OH nach der Carbodiimid-Methode, an 2-Chlortrityl-Harz durchgeführt
b) Abspaltung des Peptids von dem Harz, wobei die Schutzgruppe rBu an der L-Serin-Seitenkette verbleibt
c) Interne Zyklisierung des Peptids durch Bildung einer Esterbindung zwischen Hydroxylgruppe an der L-Threonin-Seitengruppe und der Carboxylgruppe der L-Leucin, bei dem Vorhandensein der katalytischen Mischung (DIPEA), 4-Dimethylaminopyridine und N,N'-Diisopropylcarbodiimid in den Lösungsmitteln DMF und DCM.
d) Entfernung der Schutzgruppen an der L-Serin-Seitenkette
e) Reinigung des Peptids, das in Stufe d) hergestellt wird
wobei die Synthese-Effizienz bis zu 68 % beträgt

2. Verfahren zur Synthese der Pseudofactin nach Anspruch 1, wobei die Stufe a) umfasst:
i. Koppeln der ersten Aminosäure mit Harz, wobei als Aminosäure Fmoc-L-Leu eingesetzt wird,
ii. Synthese des linearen Peptids Palm-Gly-Ser (tBu}-Thr-Leu-Leu-Ser (tBu)-Leu- Leu-OH

3. Verfahren zur Synthese der Pseudofactin nach Anspruch 1, **dadurch gekennzeichnet, dass** die Abspaltung des Peptids vom Harz, wobei die Schutzgruppe an der K-Serin-Seitenkette erhalten bleibt, mit dem Lösungsmittel, das 50 % Essigsäure und 50 % Toluol enthält, erfolgt.

## Revendications

1. Procédé de synthèse de pseudofactine comportant les étapes suivantes :
a) synthèse en phase solide du peptide Palm-Gly-Ser (tBu)-Thr-Leu-Leu-Ser (tBu)-Leu-Leu-OH par la méthode carbodiimide en utlisant la résine 2-chlorotrityle
b) clivage du peptide de la résine, en maintenant le groupe protecteur tBu sur la chaîne latérale de L-sérine
c) cyclisation interne du peptide par formation de la liaison ester entre la fonction hydroxyle sur la chaîne latérale de L-tréonine et la fonction carboxylique de L-léucine, en présence du mélange catalytique de (DIPEA), de 4- diméthylaminopyridine N, de N'-diisopropylcarbodiimide dans les solvants DMF et DCM.
d) suppression des groupes protecteurs disposés sur la chaîne latérale de L-sérine
e) lavage du peptide obtenu à l'étape d)
où le rendement de la synthèse est de 68%.

2. Procédé de synthèse de pseudofactine selon la revendication 1, dans lequel l'étape a) comprend :
i. addition du premier acide aminé à la résine, l'acide aminé étant Fmoc-L-Leu.
ii. synthèse du peptide linéaire Palm-Gly-Ser (tBu}-Thr-Leu-Leu-Ser (tBu)-Leu- Leu-OH

3. Procédé de synthèse de pseudofactine selon la revendication 1, **caractérisé en ce que** le clivage du peptide de la résine tout en maintenant simultanément le groupe protecteur dans la chaîne latérale de L-sérine est effectué avec la solution comprenant 50% d'acide acétique et 50% de toluène.
